# EUROPÄISCHE PATENTANMELDUNG

(11) **EP 3 009 114 A1**
(43) Veröffentlichungstag der Anmeldung: **20.04.2016**
(21) Anmeldenummer: 15189492.0
(22) Anmeldetag: 13.10.2015
(51) Int. Cl.: A61F 11/00, A61F 13/38

(54) **GERÄT ZUM REINIGEN VON OHREN**

(30) Priorität: 15.10.2014 DE 202014104902 U
(71) Anmelder: Janßen, Dirk, 67122 Altrip (DE); Kovacevic, Milos, 20354 Hamburg (DE); Schulz, Kai-Jörg, 68723 Schwetzingen (DE)
(72) Erfinder: Janßen, Dirk, 67122 Altrip (DE); Kovacevic, Milos, 20354 Hamburg (DE); Schulz, Kai-Jörg, 68723 Schwetzingen (DE)
(74) Vertreter: Dosterschill, Peter

(57) **Zusammenfassung**

Die Erfindung betrifft ein Gerät zum Reinigen von Ohren, das aus einem stabförmigen Trägerelement (1) mit einem ersten Endbereich und einem zweiten Endbereich besteht, und aus einem Reinigungselement, das in dem ersten Endbereich des Trägerelements (1) angeordnet ist.

Erfindungsgemäß ist vorgesehen, dass rillenartige Vertiefungen oder eine an dem stabförmigen Trägerelement (1) angeordnete Spirale (23) schräg zu dem stabförmigen Trägerelement (1) angeordnet sind, wobei die rillenartigen Vertiefungen oder die Spirale (23) eine Förderschnecke bilden.

## Beschreibung

Die Erfindung betrifft ein Gerät zum Reinigen von Ohren nach dem Oberbegriff des Patentanspruchs 1.

Zur Reinigung des menschlichen Gehörganges werden meist Ohrenstäbchen verwendet, die in der Regel als aus einem ca. 80 mm langen Rundstab oder Rohr ausgeführt in der Regel aus verpresster Cellulose oder Kunststoff und einem an einem oder beiden Enden befestigten und fest geformten, tropfenförmigen Baumwolltupfer in einer Länge von etwa 10mm und einem Maximaldurchmesser von 5 mm (Q-Tips) ausgeführt sind.

Die Benutzung dieser Art von Ohrenstäbchen kann dazu führen, das Cerumen nicht entfernt wird, sondern auch in Richtung Trommelfell geschoben und bei häufiger Anwendung dort verdichtet und verfestigt wird. Dies kann zu Beeinträchtigungen des Hörvermögens führen und das so verfestigte Cerumen ist vom Facharzt zu entfernen.

Aus DE 196 38 913 A1 ist ein Reinigungsstäbchen, insbesondere zur Ohrreinigung bekannt. Dieses besteht aus einem Stiel und wenigstens einer etwa eiförmigen Verdickung am Stielende. In die Oberfläche der Verdickung sind Längsrillen eingeformt, die sägezahnförmig ausgebildet sind.

Aus DE 10 2004 057 109 A1 ist ein Ohr-Reinigungsstäbchen bekannt, welches vorzugsweise an beiden Enden mit ballenartigen Profilkörpern ausgestattet ist. Die ballenartigen Profilkörper weisen rillenartige Vertiefungen auf, um dadurch eine schonende Schutzaufnahme im Gehörgang zu ermöglichen.

Ausgehend von diesem Stand der Technik liegt der Erfindung die Aufgabe zugrunde, ein Gerät zu schaffen, mit dem Benutzer Verschmutzungen sowohl im Gehörgang als auch am Trommelfell ihrer Ohren vollständig und in einfacher Weise entfernen.

Die Aufgabe wird durch ein Gerät gelöst, das in den Patentansprüchen definiert ist.

Das erfindungsgemäße Gerät zum Reinigen von Ohren, insbesondere des Gehörgangs besteht aus einem stabförmigen Trägerelement mit einem ersten Endbereich und einem zweiten Endbereich, aus einem Reinigungselement, das in dem ersten Endbereich des Trägerelements angeordnet ist, wobei bei einer ersten Ausführungsform des erfindungsgemäßen Geräts rillenartige Vertiefungen in dem Reinigungselement angeordnet sind, und wobei bei einer zweiten Ausführungsform des erfindungsgemäßen Geräts eine Spirale an dem Trägerelement angeordnet ist. Die rillenartigen Vertiefungen und/oder die Spirale sind schräg zu dem stabförmigen Trägerelement angeordnet und bilden eine Förderschnecke.

Damit wird der Vorteil erzielt, dass sowohl Verschmutzungen im Gehörgang als auch am Trommelfell vollständig und rasch aus dem Ohr entfernt werden.

Eine vorteilhafte Ausführungsform der Erfindung ist dadurch gekennzeichnet, dass die Förderschnecke in der Weise ausgestaltet ist, dass sie bei mindestens einer Drehung um das stabförmige Trägerelement im Gehörgang dort vorhandene Verschmutzungen vom ersten Endbereich in Richtung des zweiten Endbereichs führt. Die Förderschnecke transportiert die Verschmutzungen, ausgelöst durch Drehungen des Geräts durch den Benutzer; die Förderschnecke lässt sich zudem in einfacher Weise reinigen, beispielsweise durch einen Wasserstrahl.

Eine weitere vorteilhafte Ausführungsform der Erfindung ist dadurch gekennzeichnet, dass die Drehung im Uhrzeigersinn oder gegen den Uhrzeigersinn erfolgt.

Damit wird der Vorteil erzielt, dass ein Benutzer beispielweise sein rechtes Ohr mittels einer oder mehrerer Drehungen im Uhrzeigersinn reinigt, während er sein linkes Ohr mittels einer oder mehrerer Drehungen gegen den Uhrzeigersinn reinigt. Diese Drehungen entsprechen den natürlichen Drehbewegungen der rechten beziehungsweise der linken Hand.

Eine weitere vorteilhafte Ausführungsform der Erfindung ist dadurch gekennzeichnet, dass die Förderschnecke einen ersten Kontaktbereich aufweist, der stirnseitig zu dem stabförmigen Trägerelement angeordnet ist, sowie einen zweiten Kontaktbereich, der seitlich zu dem stabförmigen Trägerelement angeordnet ist.

Damit wird der Vorteil erzielt, dass die Förderschnecke Verschmutzungen in unterschiedlichen Bereichen des Ohrs erfasst und entfernt.

Eine weitere vorteilhafte Ausführungsform der Erfindung ist dadurch gekennzeichnet, dass die Förderschnecke im ersten Kontaktbereich schaufelförmig ausgebildet ist. Damit wird der Vorteil erzielt, dass Verschmutzungen effektiv von dem Trommelfell aufgenommen werden. Diese Ausführungsform ist insbesondere auch für medizinisches Fachpersonal geeignet, das Patienten entsprechend behandelt.

Eine weitere vorteilhafte Ausführungsform der Erfindung ist dadurch gekennzeichnet, dass das stabförmige Trägerelement ein Rohr bildet, das eine Reinigungsflüssigkeit vom zweiten Bereich zum ersten Endbereich führt.

Damit wird der Vorteil erzielt, dass cerumenlösende Flüssigkeiten gezielt durch das Rohr zum Trommelfell und/oder zu den Wandungen des Gehörganges geführt werden können. Damit lässt sich ergänzend zu dem Einsatz der Förderschnecke die Reinigung eines Ohres weiter unterstützen.

Eine weitere vorteilhafte Ausführungsform der Erfindung ist dadurch gekennzeichnet, dass das Trägerelement und/oder das Reinigungselement aus Kunststoff bestehen und/oder dass mindestens eine Komponente des Gerätes aus Baumwolle und/oder Cellulose besteht.

Damit wird der Vorteil einer kostengünstigen Herstellung erzielt.

Die Erfindung wird nun anhand der Figuren beispielhaft beschrieben.

Es zeigt
- Figur 1: einen Schnitt durch eine erste Ausführungsform des erfindungsgemäßen Geräts;
- Figur 2a: eine Seitenansicht eine zweite Ausführungsform des erfindungsgemäßen Geräts;
- Figur 2b: einen Schnitt durch die zweite Ausführungsform nach Figur 2a; und
- Figur 3: eine Seitenansicht einer Kreisbogenschaufel und einer Förderspirale der zweiten Ausführungsform nach den Figuren 2a und 2b.

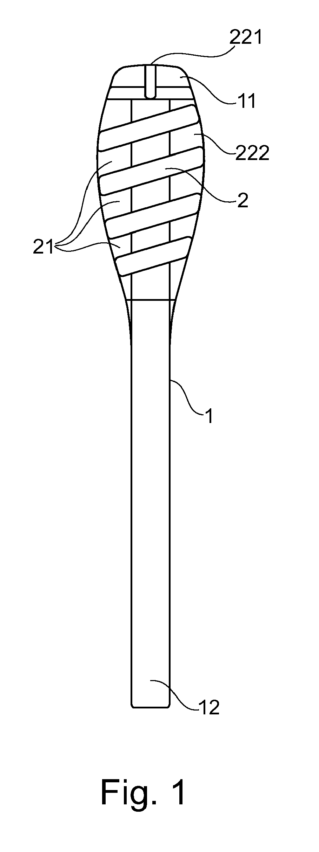

Die Figur 1 zeigt das Gerät zum Reinigen von Ohren, insbesondere des Gehörgangs, welches ein stabförmiges Trägerelement 1 mit einem ersten Endbereich 11 und einem zweiten Endbereich 12 aufweist. Im ersten Endbereich ist ein Reinigungselement 2 angeordnet, das aus einer Förderschnecke besteht. Diese weist rillenartige Vertiefungen 21 auf, die schräg zu dem stabförmigen Trägerelement angeordnet sind.

Die Förderschnecke ist in der Weise ausgestaltet, dass sie bei mindestens einer Drehung um das stabförmige Trägerelement 1 im Gehörgang dort vorhandene Verschmutzungen (Cerumen, Ohrenschmalz) vom ersten Endbereich 11 in Richtung des zweiten Endbereichs 12 führt. Die jeweilige Drehung erfolgt im Uhrzeigersinn oder gegen den Uhrzeigersinn.

Die Förderschnecke weist einen ersten Kontaktbereich 221 zu dem Innenohr auf, der stirnseitig zu dem stabförmigen Trägerelement 1 angeordnet ist. Daneben weist die Förderschnecke einen zweiten Kontaktbereich 222 auf, der seitlich zu dem stabförmigen Trägerelement 1 angeordnet ist.

Im ersten Kontaktbereich 221 ist die Förderschnecke schaufelförmig ausgebildet, wobei keine scharfen Kanten vorgesehen sind, die bei unsachgemäßer Handhabung des Geräts zu Verletzungen führen könnten. Vielmehr sind bei dem erfindungsgemäßen Gerät Schaufelkanten abgerundet ausgebildet. Diese lösen die zuvor genannten Verschmutzungen ab. Auch die Förderschnecke entfernt Verschmutzungen aus dem Ohr.

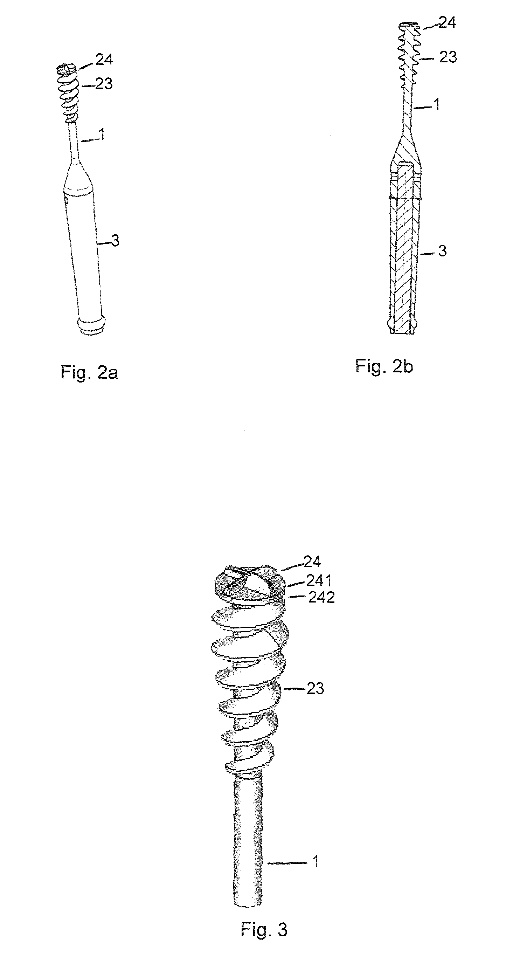

Die in den Figuren 2a, 2b und 3 dargestellte Ausführungsform weist keine rillenartigen Vertiefungen (21) auf, sondern eine Förderspirale 23, die an dem Trägerelementenkörper anliegt und damit schräg zu dem stabförmigen Trägerelement 1 angeordnet ist. Die Förderspirale 23 bildet bei dieser Ausführungsform die Förderschnecke.

Die Förderschnecke ist im ersten Kontaktbereich (221 in Figur 1) schaufelförmig ausgebildet und weist im ersten Kontaktbereich eine Kreisbogenschaufel 24 oder eine logarithmische Schaufel auf.

Die jeweilige Schaufel weist vorzugsweise eine kreisförmige Grundfläche 241 auf, wobei an der kreisförmigen Grundfläche 241 eine Platte 242 angeordnet sein kann. Die Platte 242 kann die Grundfläche der jeweiligen Schaufel bilden.

Während mit der Schaufel 24 (Figuren 2a, 2b, 3) Cerumen im Bereich des Trommelfells entfernt wird, dienen die rillenartigen Vertiefungen 21 (Figur 1) und die Förderspirale 23 (Figuren 2a, 2b, 3) dazu, Cerumen im Gehörgang zu entfernen, wo sich üblicherweise der Großteil des zu entfernenden Cerumens befindet.

Anstelle der Schaufel 24 kann der erste Kontaktbereich (221 in Figur 1) auch kugelförmig ausgebildet sein und beispielsweise die Form eines Kugelstumpfs aufweisen.

Weiterhin kann das Gerät einen insbesondere ergonomisch optimierten Handgriff 3 aufweisen, in den das stabförmige Trägerelement 1 einsetzbar ist.

Der Handgriff 3 kann einen Innenraum für die Aufnahme eines Ersatz-Trägerelements 1 aufweisen. Im Innenraum des Handgriffs 3 kann auch ein Elektromotor sowie eine Batterie für dessen Aktivierung angeordnet sein. Der Elektromotor ist mit dem Trägerelement 1 verbunden, der dieses in Drehbewegungen versetzt. Hierzu ist ein entsprechender elektrischer Schalter am Handgriff 3 angeordnet.

Der Handgriff 3 kann auch mindestens eine Öffnung für die Zuleitung einer nachstehend beschriebenen Reinigungsflüssigkeit aufweisen.

Der Handgriff 3 weist beispielsweise eine Elastomer-Ummantelung auf.

In einer bevorzugten Ausführungsform des erfindungsgemäßen Geräts bildet das stabförmige Trägerelement 1 ein Rohr, in das im zweiten Bereich 12 eine Reinigungsflüssigkeit eingeführt und zum ersten Endbereich 11 geführt wird. Im ersten Endbereich 11 des stabförmigen Trägerelements 1 befinden sich Öffnungen, durch die die Reinigungsflüssigkeit unter einem vorgebbaren Druck austritt und an den Gehörgang gelangt. Auf diese Weise wird eine cerumenlösende Flüssigkeit gezielt durch das Rohr zu den Wandlungen des Gehörganges und/oder zum Trommelfell geführt. Hierzu weist das Gerät in seinem zweiten Endbereich 12 einen Anschluss für eine Pumpspray-Einrichtung oder für eine Einzeldosispipette auf.

Im zweiten Endbereich 12 befindet sich ein in Figur 1 nicht dargestellter Knauf, der dem Benutzer die korrekte Drehrichtung des Gerätes anzeigt oder auch durch eine Rasteinrichtung vorgibt.

Das Trägerelement 1 und/oder das Reinigungselement 2 bestehen aus Kunststoff, insbesondere aus weichem medizinischen Kunststoff, beziehungsweise aus Silikon oder einem thermoplastischen Elastomer. Mindestens eine Komponente des Gerätes kann aus Baumwolle und/oder aus Cellulose bestehen.

Das stabförmige Trägerelement 1 hat vorzugsweise eine Länge im Bereich von 80 mm sowie einen Durchmesser vorzugsweise im Bereich von 4 mm. Die Vertiefungen 21 sind vorzugsweise um 2,5 mm beabstandet. Die Förderschnecke hat beispielsweise einen Querschnitt in Form eines Ellipsenabschnittes, vorzugsweise eine Länge im Bereich von 15 mm und vorzugsweise eine Breite im Bereich von 5 mm.

### Bezugszeichenliste

- 1: Trägerelement
- 11: Erster Endbereich
- 12: Zweiter Endbereich

- 2: Reinigungselement
- 21: Vertiefung
- 221: Erster Kontaktbereich
- 222: Zweiter Kontaktbereich
- 23: Spirale, Förderspirale
- 24: Schaufel; Kreisbogenschaufel, logarithmische Schaufel
- 241: Grundfläche der Schaufel
- 242: Platte

- 3: Handgriff

## Patentansprüche

1. Gerät zum Reinigen von Ohren, bestehend aus
- einem stabförmigen Trägerelement (1) mit einem ersten Endbereich (11) und einem zweiten Endbereich (12),
- einem Reinigungselement (2), das in dem ersten Endbereich des Trägerelements (1) angeordnet ist,
- rillenartigen Vertiefungen (21), die in dem Reinigungselement (2) angeordnet sind,
**dadurch gekennzeichnet,**
- **dass** die rillenartigen Vertiefungen (21) oder eine an dem stabförmigen Trägerelement (1) angeordnete Spirale (23) schräg zu dem stabförmigen Trägerelement (1) angeordnet sind, und
- **dass** die rillenartigen Vertiefungen (21) oder die Spirale (23) eine Förderschnecke bilden.

2. Gerät nach Anspruch 1, **dadurch gekennzeichnet,**
- **dass** die Förderschnecke in der Weise ausgestaltet ist, dass sie bei mindestens einer Drehung um das stabförmige Trägerelement (1) im Inneren eines Ohres dort vorhandene Verschmutzungen vom ersten Endbereich (11) in Richtung des zweiten Endbereichs (12) führt.

3. Gerät nach Anspruch 2, **dadurch gekennzeichnet,**
- **dass** die Drehung im Uhrzeigersinn oder gegen den Uhrzeigersinn erfolgt.

4. Gerät nach einem der vorstehenden Ansprüche, **dadurch gekennzeichnet,**
- **dass** die Förderschnecke einen ersten Kontaktbereich (221) aufweist, der stirnseitig zu dem stabförmigen Trägerelement (1) angeordnet ist, sowie einen zweiten Kontaktbereich (222), der seitlich zu dem stabförmigen Trägerelement (1) angeordnet ist.

5. Gerät nach Anspruch 4, **dadurch gekennzeichnet,**
- **dass** die Förderschnecke im ersten Kontaktbereich (221) schaufelförmig oder kugelförmig ausgebildet ist.

6. Gerät nach Anspruch 5, **dadurch gekennzeichnet,**
- **dass** die Förderschnecke im ersten Kontaktbereich (221) eine Kreisbogenschaufel (24) oder eine logarithmische Schaufel aufweist.

7. Gerät nach Anspruch 6, **dadurch gekennzeichnet,**
- **dass** die Kreisbogenschaufel (24) oder die logarithmische Schaufel eine kreisförmige Grundfläche (241) aufweist, und
- **dass** an der kreisförmigen Grundfläche (241) eine Platte (242) angeordnet ist.

8. Gerät nach einem der vorstehenden Ansprüche, **dadurch gekennzeichnet,**
- **dass** es einen Handgriff (3) aufweist, in den das stabförmige Trägerelement (1) einsetzbar ist.

9. Gerät nach Anspruch 8, **dadurch gekennzeichnet,**
- **dass** der Handgriff (3) einen Innenraum für die Aufnahme eines Ersatz-Trägerelements (1) und/oder für die Aufnahme eines Elektromotors und einer Batterie aufweist.

10. Gerät nach Anspruch 8 oder 9, **dadurch gekennzeichnet,**
- **dass** der Handgriff (3) eine Elastomer-Ummantelung aufweist.

11. Gerät nach einem der vorstehenden Ansprüche, **dadurch gekennzeichnet,**
- **dass** das stabförmige Trägerelement (1) anstelle der Förderschnecke oder zusätzlich zu der Förderschnecke ein Rohr bildet, das eine Reinigungsflüssigkeit vom zweiten Endbereich (12) zum ersten Endbereich (11) führt.

12. Gerät nach einem der vorstehenden Ansprüche, **dadurch gekennzeichnet,**
- **dass** das Trägerelement (1) und/oder das Reinigungselement (2) aus Kunststoff bestehen.

13. Gerät nach einem der vorstehenden Ansprüche, **dadurch gekennzeichnet,**
- **dass** mindestens eine Komponente des Gerätes aus Baumwolle und/oder Cellulose besteht.
